(19) Europäisches Patentamt
European Patent Office
Office européen des brevets

(11) **EP 4 417 700 A1**

(12) **EUROPEAN PATENT APPLICATION**
published in accordance with Art. 153(4) EPC

(43) Date of publication:
**21.08.2024 Bulletin 2024/34**

(21) Application number: **22880795.4**

(22) Date of filing: **29.09.2022**

(51) International Patent Classification (IPC):
***C12Q 1/06*** (2006.01)   ***C12M 1/34*** (2006.01)

(52) Cooperative Patent Classification (CPC):
**C12M 1/34; C12Q 1/06**

(86) International application number:
**PCT/JP2022/036466**

(87) International publication number:
**WO 2023/063099 (20.04.2023 Gazette 2023/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.10.2021 JP 2021168634**

(71) Applicants:
• **Shimadzu Corporation**
  **Nakagyo-ku,**
  **Kyoto-shi,**
  **Kyoto 604-8511 (JP)**
• **Kyoto University**
  **Kyoto-shi, Kyoto 606-8501 (JP)**

• **Koga Fumitoshi Women's Clinic**
  **Fukuoka-shi, Fukuoka 810-0001 (JP)**

(72) Inventors:
• **TANAKA, Motomu**
  **Kyoto-shi, Kyoto 606-8501 (JP)**
• **YAMAMOTO, Akihisa**
  **Kyoto-shi, Kyoto 606-8501 (JP)**
• **SUGIMURA, Kaori**
  **Kyoto-shi, Kyoto 604-8511 (JP)**
• **KOGA, Fumitoshi**
  **Fukuoka-shi, Fukuoka 810-0001 (JP)**
• **KITAKAMI, Shigeki**
  **Fukuoka-shi, Fukuoka 810-0001 (JP)**

(74) Representative: **Müller-Boré & Partner**
**Patentanwälte PartG mbB**
**Friedenheimer Brücke 21**
**80639 München (DE)**

(54) **OVUM EVALUATION METHOD, OVUM EVALUATION DEVICE, AND PROGRAM FOR EVALUATING OVUM**

(57)    One mode of an ovum evaluation method according to the present invention is a method for evaluating an ovum, the method including: an analysis step (S21 to S24) of analyzing a deformation state of a target ovum using an image obtained by photographing the ovum to non-invasively obtain an index value obtained by quantifying a mechanical property of the ovum; and an evaluation step (S25) of evaluating a quality of the target ovum on the basis of the index value obtained in the analysis step. As a result, the quality of the ovum can be evaluated accurately according to theoretical criteria on the basis of the result of non-invasive measurement or observation.

Fig. 2

OVUM EVALUATION PROCESSING BEFORE INSEMINATION

S20  PHOTOGRAPH TARGET OVUM OVER PREDETERMINED TIME

S21  EXTRACT CONTOUR OF OVUM IN EACH FRAME IMAGE CONSTITUTING ACQUIRED MOVING IMAGE

S22  CALCULATE RADIUS FROM CONTOUR OF OVUM FOR EACH FRAME IMAGE

S23  CALCULATE RADIUS CHANGE AMOUNT OF OVUM

S24  CALCULATE THREE TYPES OF MECHANICAL PARAMETERS BY FITTING RADIUS CHANGE AMOUNT OF OVUM TO THEORETICAL FORMULA

S25  DERIVE INFORMATION ON QUALITY OF OVUM USING LEARNED MODEL IN WHICH THREE TYPES OF MECHANICAL PARAMETERS ARE USED AS EVALUATION INDEX

END

EP 4 417 700 A1

**Description**

TECHNICAL FIELD

The present invention relates to a method, an apparatus, and a computer program for evaluating the quality of an ovum.

BACKGROUND ART

In vitro fertilization and intracytoplasmic sperm injection are well known as clinical treatments of assisted reproductive technology in humans. In vitro fertilization is a method in which an ovum taken out from a patient is mixed with sperm (inseminated) to promote fertilization, and the fertilized ovum is cultured for a predetermined period and then transferred to a mother's body (embryo transfer). On the other hand, intracytoplasmic sperm injection is a method in which a fine glass needle called an injection pipette is inserted into an ovum taken out from a patient, and one sperm is injected into the ovum by the injection pipette to perform fertilization. This intracytoplasmic sperm injection is performed by a professional technician (commonly referred to as "embryologist") under microscopic observation.
In the case of intracytoplasmic sperm injection, fertilization is artificially performed by an embryologist. Thus, in order to increase a fertilization rate and an implantation rate after embryo transfer, it is very important to choose a mature and high-quality ovum from a plurality of ova retrieved from a patient and perform insemination. To choose such a good-quality ovum is also one of important tasks for an embryologist. In general, evaluation of the quality of an ovum and choosing of an ovum based on the evaluation result have conventionally been dependent mostly on the manual work of an embryologist. For this reason, it is inevitable that the result varies due to a difference in skill such as the experience and skill level of an embryologist in charge. In addition, such a work thus imposes a large load on the embryologist, and it is difficult to increase efficiency (throughput). Furthermore, in many cases, the quality of an ovum is not evaluated according to clear and quantitative criteria, which makes it difficult to verify or check whether the evaluation is appropriate.
The fact that the quality of an ovum is related to the hardness of the ovum is well-known knowledge based on the experiences of embryologists. Therefore, for the purpose of evaluating the quality of an ovum, a technique for measuring the degree of softness and hardness of an ovum has been previously proposed.
For example, Patent Literature 1 discloses a technique for quantitatively measuring hardness, which is one of mechanical features of an ovum, from deformation responsiveness, a strain state, a deformation state, or the like of an outer membrane based on local deformation of the ovum observed in an image obtained by photographing the ovum in a state of being pinched by a microprobe.
Non Patent Literature 1 mentions that a human embryo that is too hard or too soft when sucked by a manipulator has poor developmental potential.
Non Patent Literature 2 discloses a technique in which a micro force sensor using a strain gauge is attached to the tip of a two-fingered micro hand, and measures a reaction force generated at an end effector portion when the two-fingered micro hand grips a cell, to estimate the stiffness of the cell.
Non Patent Literature 3 mentions that it has been confirmed that the elasticity of the zona pellucida of an ovum at each stage of ovum maturation, fertilization, and early embryo development specifically changes by using a micro tactile sensor system having a strain gauge at the base of a needle.

CITATION LIST

PATENT LITERATURE

Patent Literature 1: JP 2016-54684 A

NON PATENT LITERATURE

Non Patent Literature 1: Livia Z. Yanez and 4 other persons, "Human oocyte developmental potential is predicted by mechanical properties within hours after fertilization", Nature Communications, Vol. 7, No. 1, 2016, pp. 1-12
Non Patent Literature 2: "Force measurement in microenvironment using two-fingered micro hand (Arai Laboratory, Osaka University)", [Online], [Retrieved January 22, 2021], Internet <URL: http://www.arailab.sys.es.osaka-u.ac.jp/index.php?option=com_content&view=article&id=75&Itemid=97&lang=ja>
Non Patent Literature 3: Murayama Yoshinobu and 9 other persons, "Elasticity measurement of zona pellucida using a micro tactile sensor to evaluate embryo quality", Journal of Mammalian Ova Research, Vol. 25, No. 1, 2008, pp. 8-16

Non Patent Literature 4: Ito Hiroaki and 7 other persons, "Quantification of the influence of endotoxins on the mechanics of adult and neonatal red blood cells", The Journal of Physical Chemistry B, Vol. 119, No. 25, 2015, pp. 7837-7845

## SUMMARY OF INVENTION

### TECHNICAL PROBLEM

However, in any of the conventional techniques as described above, an invasive method is used to acquire information related to the hardness or elasticity of an ovum for the purpose of evaluating the quality of the ovum. In intracytoplasmic sperm injection, temporarily holding an ovum and inserting an injection pipette into the ovum at the time of insemination are essential works for treatment. However, performing an invasive operation on the ovum for other works has a risk of damaging the ovum, which is not preferable. In addition, in the method described in Patent Literature 1, it is necessary to incorporate an ovum into a dedicated chip and pinch the ovum, which takes extra time and effort that are not required in a normal treatment process. There is also a concern that a prolonged work time further damages the ovum.

In addition, when an ovum is pinched by a sensor, the force may be applied obliquely if the contact points of the sensor deviate from positions facing each other across the center point of the ovum because an ovum is substantially spherical, and the elasticity or the like cannot be accurately measured. In addition, since the sensor does not always contact the same position of the ovum, reproducibility in performing repeated measurements is low. Furthermore, because the ovum is usually not perfectly spherical but somehow distorted, it is quite difficult to bring the sensor into contact on an axis passing through the center point of the ovum.

On the other hand, as a method for non-invasively evaluating the quality of an ovum, a system has recently been developed using a machine learning method such as deep learning, in which an image obtained by photographing an ovum is input, and an evaluation result of the quality of an ovum is output. However, in such a method, the process of evaluating the quality of the ovum is so-called black-boxed. It cannot be said that the method is theoretical ovum quality evaluation according to clear and quantitative criteria. In this respect, the method is not so different from conventional evaluation of the quality of an ovum that relies on the knowledge and experience of an embryologist.

The present invention has been made to solve such problems, and a main object of the present invention is to provide an ovum evaluation method, an ovum evaluation apparatus, and an ovum evaluation program capable of evaluating the quality of an ovum accurately according to theoretical criteria on the basis of the result of non-invasive measurement or observation.

### SOLUTION TO PROBLEM

One mode of an ovum evaluation method according to the present invention made to solve the above problems is a method for evaluating an ovum, the method including:

an analysis step of analyzing a deformation state of a target ovum using an image obtained by photographing the target ovum to non-invasively obtain an index value obtained by quantifying a mechanical property of the target ovum; and
an evaluation step of evaluating a quality of the target ovum on the basis of the index value obtained in the analysis step.

One mode of an ovum evaluation apparatus according to the present invention made to solve the above problems is an apparatus for performing the ovum evaluation method of the above mode according to the present invention, the apparatus including:

an analyzer configured to receive an image obtained by photographing a target ovum, and analyze a deformation state of the target ovum using the image to non-invasively obtain an index value obtained by quantifying a mechanical property of the target ovum; and
an evaluator configured to evaluate a quality of the target ovum on the basis of the index value obtained by the analyzer, and output an evaluation result.

One mode of an ovum evaluation program according to the present invention made to solve the above problems is a program for evaluating an ovum using a computer, the program causing a computer to execute:

an analysis step of analyzing a deformation state of a target ovum using an image obtained by photographing the target ovum to non-invasively obtain an index value obtained by quantifying a mechanical property of the target ovum; and

an evaluation step of evaluating a quality of the target ovum on the basis of the index value obtained in the analysis step.

Here, the "target ovum" is a subject to be fertilized by in vitro fertilization or intracytoplasmic sperm injection, and is further used for performing embryo transfer through embryo culture after fertilization. For this reason, a work or an operation inevitably performed on the ovum in such assisted reproductive technology, which is a work or an operation in direct contact with the ovum, such as holding of the ovum or insertion of an injection pipette for insemination, performed in, for example, intracytoplasmic sperm injection, does not fall under an invasive work or operation in the present specification and the present invention.

In addition, in the present invention, the "image" obtained by photographing the target ovum may be an image at a certain time point, that is, a still image, or may be a moving image or time-lapse images composed of a plurality of temporally consecutive images. Therefore, the "deformation state of the target ovum" may be the state of the ovum at a certain time point when the ovum is deformed, or may be the behavior of the ovum during a deformation process of the ovum.

In addition, in the present invention, the evaluation result by the "evaluation of the quality of the ovum" can include a result indicating whether the quality of one ovum is good or not, a result of ranking the qualities of a plurality of ova, a multi-grade evaluation result corresponding to grade classification used for known evaluation of a fertilized ovum such as Veeck's classification and Gardner's classification, and a determination result indicating whether embryo transfer is suitable or not or whether cryopreservation is suitable or not. In the present invention, such an evaluation result is output as a result of processing in an apparatus (which may include a computer). The evaluation result can correspond to a decision result by an expert embryologist having a high level of skill.

In addition, the ovum evaluation program of the above mode according to the present invention can be provided to a user by being stored in a non-transitory computer-readable recording medium such as a CD-ROM, a DVD-ROM, a memory card, or a USB memory (dongle). In addition, the program can be provided to the user in the form of data transfer via a communication line such as the Internet. Furthermore, the program can be pre-installed in a computer which is a part of the system (strictly, a storage device which is a part of the computer) when the user purchases the system.

ADVANTAGEOUS EFFECTS OF INVENTION

According to the ovum evaluation method, apparatus, and program of the above mode according to the present invention, the quality of the target ovum can be non-invasively evaluated accurately on the basis of the index value obtained by quantifying the mechanical property, that is, with theoretical grounding. As a result, the ovum is not damaged during evaluation of the quality of the ovum. For example, the ovum after the evaluation can be favorably used for the original purpose. In addition, the present invention reduces the workload of an embryologist, and eliminates a variation in quality evaluation depending on the skill of an embryologist in charge, to improve the reliability of the quality evaluation of the ovum. Furthermore, in the present invention, data in evaluating the quality of the ovum remain as a numerical value, which enables an embryologist or the like to easily and objectively verify whether or not the evaluation is appropriate later.

BRIEF DESCRIPTION OF DRAWINGS

[Fig. 1] Fig. 1 is a flowchart illustrating an example of a work/processing procedure in intracytoplasmic sperm injection using an embodiment of an ovum evaluation method according to the present invention.

[Fig. 2] Fig. 2 is a flowchart illustrating a detailed processing procedure of ovum evaluation processing before insemination in the work/processing procedure of intracytoplasmic sperm injection illustrated in Fig. 1.

[Fig. 3] Fig. 3 is a schematic configuration diagram of an example of an ovum evaluation apparatus for performing ovum evaluation processing before insemination and ovum evaluation processing during insemination.

[Fig. 4] Fig. 4 is a drawing illustrating an example of an image obtained by photographing an ovum in the ovum evaluation apparatus.

[Fig. 5] Fig. 5 is a drawing illustrating an actual measurement result of a luminance profile on line U illustrated in Fig. 4.

[Fig. 6] Figs. 6A-6C are explanatory views of a process of averaging luminance values of points on line U.

[Fig. 7] Fig. 7 is a drawing illustrating a relationship between an angle $\varphi$ and a radius $r(\varphi)$ of an ovum.

[Fig. 8] Fig. 8 is a drawing illustrating a mean square amplitude (MSA) value of a radius calculated from actual measurement data and a fitting curve using a theoretical formula.

[Fig. 9] Fig. 9 is an explanatory view of a method for calculating an aspect ratio of a deformed ovum at the time of insertion of an injection pipette.

[Fig. 10] Figs. 10A and 10B are drawings illustrating a deformation state of an ovum at the time of fixation by a holding pipette.

[Fig. 11] Fig. 11 is an explanatory view on how to obtain an index value of deformation relaxation of an ovum after a time point of opening a hole by insertion of an injection pipette.

[Fig. 12] Fig. 12 is a drawing illustrating a temporal change in deformation relaxation of an ovum after a time point of opening a hole by insertion of an injection pipette.

[Fig. 13] Figs. 13A and 13B are explanatory views on how to obtain the internal area of an ovum at the time of insertion of an injection pipette.

DESCRIPTION OF EMBODIMENTS

An embodiment of an ovum evaluation method and an ovum evaluation apparatus according to the present invention will be described with reference to the accompanying drawings.

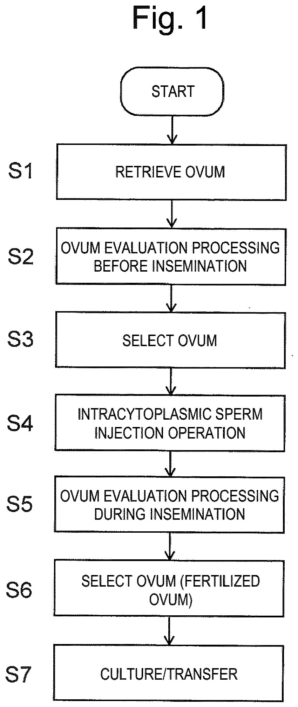

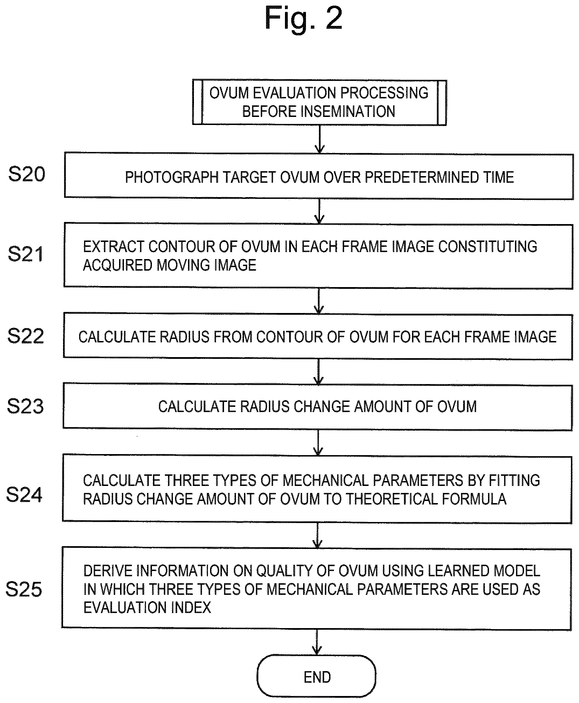

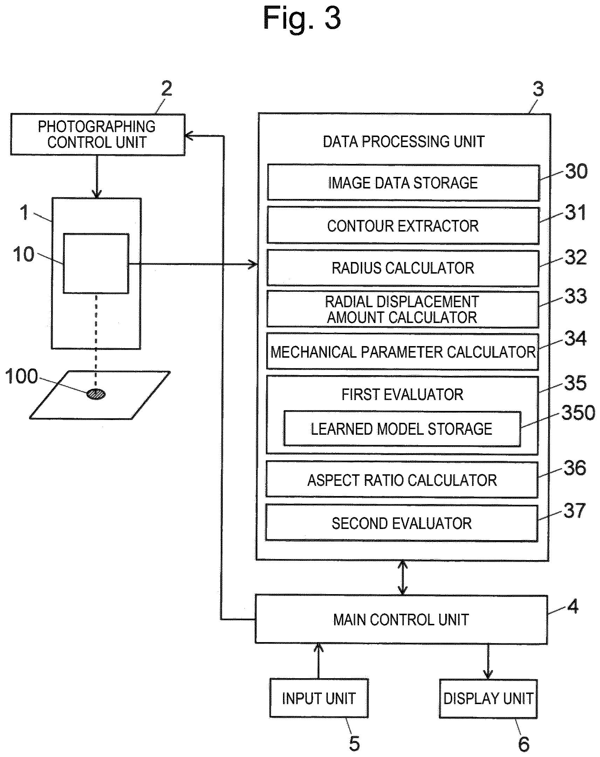

Fig. 1 is a flowchart illustrating a series of work/processing procedure in intracytoplasmic sperm injection using the embodiment of the ovum evaluation method according to the present invention. Fig. 2 is a flowchart illustrating a detailed processing procedure of ovum evaluation processing before insemination in the work/processing procedure of intracytoplasmic sperm injection illustrated in Fig. 1. Fig. 3 is a schematic configuration diagram of the embodiment of the ovum evaluation apparatus according to the present invention for performing ovum evaluation processing before insemination and ovum evaluation processing during insemination illustrated in Fig. 1.

The ovum evaluation method and the ovum evaluation apparatus of the present embodiment are mainly for evaluating the quality of an ovum and choosing a high-quality ovum in performing intracytoplasmic sperm injection which is one of assisted reproductive technologies. However, some approaches of the ovum evaluation method of the present embodiment can also be used to evaluate and choose an ovum in order to perform in vitro fertilization instead of intracytoplasmic sperm injection.

When intracytoplasmic sperm injection is performed, first, a doctor performs ovum retrieval from a patient. Usually, a plurality of ova are retrieved from the patient (step S 1).

Subsequently, an embryologist collectively or individually sets the plurality of retrieved ova in the ovum evaluation apparatus, which will be described later, and performs a predetermined operation in the apparatus. In response to this operation, the ovum evaluation apparatus performs ovum evaluation processing before insemination as first-stage ovum evaluation on the plurality of ova (step S2). The embryologist selects one or more ova to be subjected to an intracytoplasmic sperm injection work on the basis of the evaluation result by the ovum evaluation apparatus (step S3).

The embryologist performs an insemination work while observing the selected ovum with a microscope. The above ovum evaluation apparatus photographs a moving image of the state of the ovum during the insemination work (step S4).

Subsequently, the embryologist performs a predetermined operation in the above ovum evaluation apparatus. In response to this operation, the ovum evaluation apparatus performs ovum evaluation processing during insemination as second-stage ovum evaluation on the basis of the image photographed during the insemination work (step S5). Then, the embryologist selects one or more ova (fertilized ova) to be cultured (or cryopreserved) on the basis of the evaluation result by the ovum evaluation apparatus (step S6). After that, the embryologist cultures the selected fertilized ovum (ova) for a predetermined period, and after the culture, the doctor transfers an embryo obtained by the culture to the patient (step S7). Alternatively, the embryologist cryopreserves the fertilized ovum (ova) after being cultured for a predetermined period.

As described above, in the ovum evaluation method of the present embodiment, the quality of the ovum is evaluated in each of two stages of before insemination and during insemination using the ovum evaluation apparatus, which secures the quality of the fertilized ovum to be transferred to the patient. Both of these two stages of ovum evaluation are evaluation before the fertilized ovum is accommodated in an incubator, and it is possible to choose an ovum having a high fertilization success rate at an early stage. As a result, a culture loss can be reduced by efficiently choosing the fertilized ovum to be accommodated in the incubator having a limited capacity. In addition, the turnover rate of the incubator can be improved to reduce the number of standby patients for infertility treatment.

Next, the configuration of the ovum evaluation apparatus illustrated in Fig. 3 used for evaluating the quality of an ovum will be described.

The ovum evaluation apparatus includes a microscopic observation unit 1 including an image acquiring unit 10, a photographing control unit 2, a data processing unit 3, a main control unit 4, an input unit 5, and a display unit 6.

The data processing unit 3 includes, as functional blocks, an image data storage 30, a contour extractor 31, a radius calculator 32, a radial displacement amount calculator 33, a mechanical parameter calculator 34, a first evaluator 35, an aspect ratio calculator 36, and a second evaluator 37. As will be described in detail later, the first evaluator 35 performs classification or regression by machine learning, and includes a learned model storage 350 that stores a learned model trained in advance using training data for this purpose.

The microscopic observation unit 1 may be either a bright field microscope or a phase contrast microscope. The image acquiring unit 10 may acquire either a color image or a monochrome image. In addition, the image acquiring unit 10 may be a video camera capable of photographing a moving image at a general frame rate (60 frames/second), or may be a camera that performs time-lapse imaging at predetermined time intervals at a moderately reduced frame rate.

At least some functions of the data processing unit 3 and the main control unit 4 can include a computer such as a personal computer as a hardware resource, and control/processing software (program) installed in advance in the computer is executed on the computer to implement operations of the above respective functional blocks. In this case, the input unit 5 and the display unit 6 are a keyboard or a pointing device (such as a mouse), and a monitor, respectively, attached to the personal computer. This computer program is an embodiment of an ovum evaluation program according to the present invention.

Next, with reference to Fig. 2, a description will be given of a work performed by the embryologist and a processing operation by the above ovum evaluation apparatus in performing the processing in step S2 in Fig. 1, that is, the ovum evaluation processing before insemination using the ovum evaluation apparatus.

The embryologist (or another person in charge) sets a target ovum 100 to be evaluated on a stage of the microscopic observation unit 1 so that its polar body does not appear in the image, and performs an operation of giving an instruction to start analysis from the input unit 5. In response to this instruction, the photographing control unit 2 operates the image acquiring unit 10 to photograph a moving image of the ovum 100 over a predetermined time (for example, about 30 seconds) (step S20). The moving image data obtained by the image acquiring unit 10 is transferred to the data processing unit 3 and temporarily stored in the image data storage 30.

Subsequently, in the data processing unit 3, the contour extractor 31 executes a process of extracting the contour of the ovum in each frame image of the moving image (step S21). Specifically, the contour extractor 31 performs the following processing.

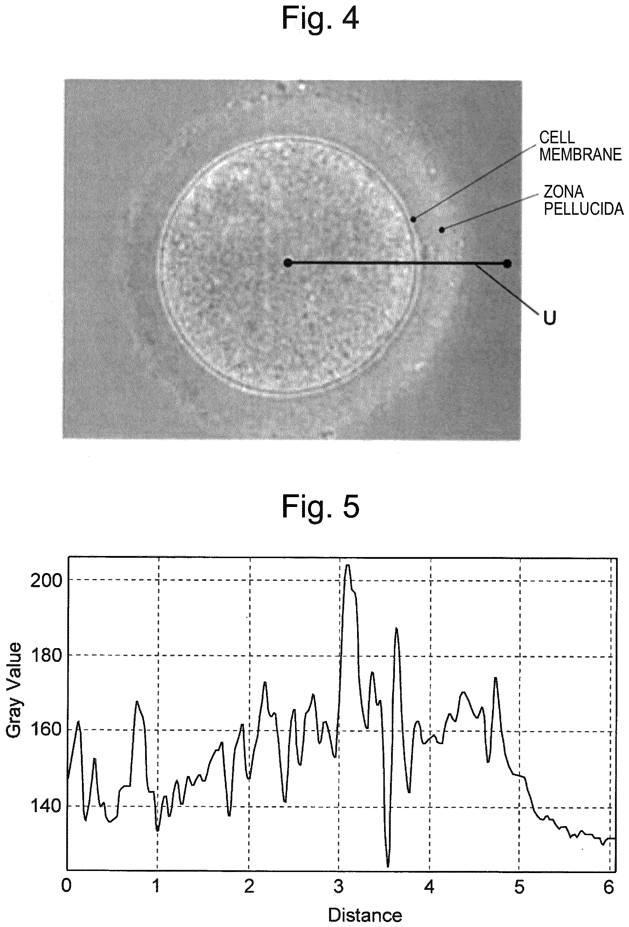

The contour extractor 31 first performs noise removal processing using a Gaussian filter, a median filter, or the like as preprocessing on each frame image. Fig. 4 is an example of the photographed image of the ovum. The ovum (egg cell) is covered with a cell membrane, and one type of protective layer called a zona pellucida exists outside the cell membrane. The above noise removal processing is processing for removing fine salt-and-pepper noise generated in the vicinity of the zona pellucida and the cell membrane on the image.

The contour extractor 31 subsequently obtains, for each frame image, a luminance profile indicating a change in luminance values of pixels along a straight line extending outward (or pixels at predetermined intervals) from the center point of the ovum. Fig. 5 is a luminance profile on straight line U illustrated in Fig. 4. Here, the center point of the ovum can be obtained by, for example, approximation of center coordinates of a circle using a least squares method (by calculating the center of a circle from contour coordinates).

In order to obtain a clear image with suppressed noise, the ovum may be irradiated with strong illumination light. However, such strong light may damage the ovum. For this reason, it is necessary to reduce the illumination light when photographing the ovum. As a result, noise (luminance noise) caused by photographing under poor illumination is conspicuous in the luminance profile, and this noise can be an error factor in extracting the contour from the luminance profile. Therefore, in order to eliminate the luminance noise, the contour extractor 31 executes smoothing processing in which the luminance value of each point (pixel) on the luminance profile is replaced with a value obtained by averaging the luminance values of a total of five points including two points in front of the point and two points behind the point.

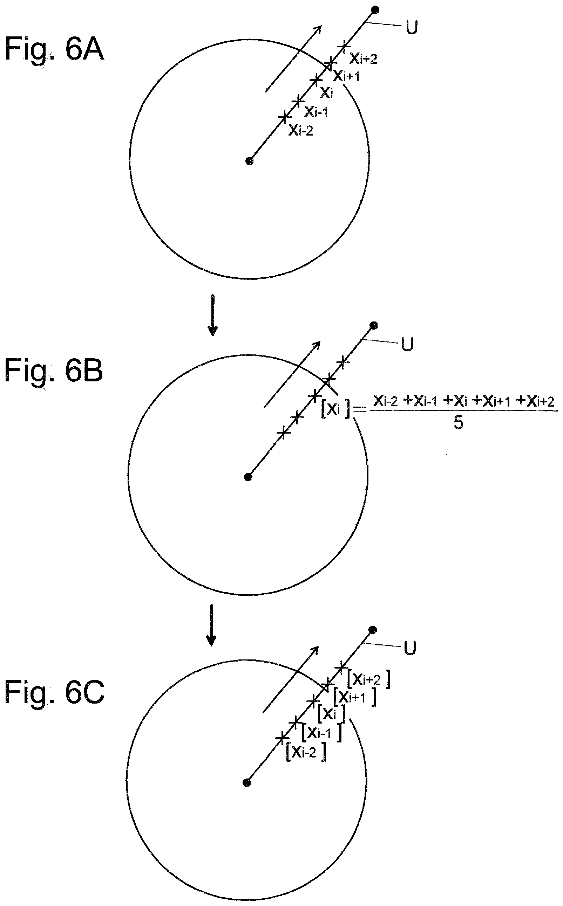

Figs. 6A-6C are explanatory views of the smoothing processing. Now, when the luminance values of five consecutive points on straight line U are $x_{i-2}$, $x_{i-1}$, $x_i$, $x_{i+1}$, and $x_{i+2}$ as illustrated in Fig. 6A, the luminance value of one point in the middle among the five points is calculated by $[x_i] = (x_{i-2} + x_{i-1} + x_i + x_{i+1} + x_{i+2})/5$ as illustrated in Fig. 6B. By performing similar calculation for each point, the respective luminance values of the above five points are replaced with $[x_{i-2}]$, $[x_{i-1}]$, $[x_i]$, $[x_{i+1}]$, and $[x_{i+2}]$ as illustrated in Fig. 6C. As a result, it is possible to obtain a luminance profile in which the influence of the luminance noise is reduced.

After that, the contour extractor 31 selects a maximum value (or a minimum value) among differential values as a point on the contour in the luminance profile after the smoothing processing along a plurality of straight lines extending radially in different directions from the center point of the ovum. The contour of the target ovum can be accurately extracted by obtaining the point on the contour in each of the straight lines drawn at predetermined angular intervals in an angular range of 360° around the center point of the ovum, and connecting the points together.

Non Patent Literature 4 written by some of the present inventors discloses that the contour of a red blood cell having a shape close to a circle is extracted from an image of the red blood cell to obtain a radius (a distance from the center point to the contour). The present inventors have studied whether the same method can be applied to an ovum. However, it is difficult to directly apply the same method. The reason is that, in the case of a red blood cell, the curve shape of the luminance profile is smooth and the contour appears quite clearly, whereas in the case of an ovum, a large amount of noise is included particularly around the cell membrane due to a thick zona pellucida existing outside the cell membrane, which makes it difficult to extract the contour.

Therefore, the contour extractor 31 first calculates the luminance profiles along radial straight lines in 64 directions at $(360/64)°$ angular intervals around the center point of the ovum, and roughly extracts a range corresponding to the contour in the luminance profiles. After that, while gradually narrowing a calculation range of the luminance profiles in the radial direction to a range estimated to include the contour, the contour extractor 31 repeats similar calculation 3 times along radial straight lines in 256 directions at $(360/256)°$ angular intervals around the center point of the ovum, to finally obtain the contour with high accuracy.

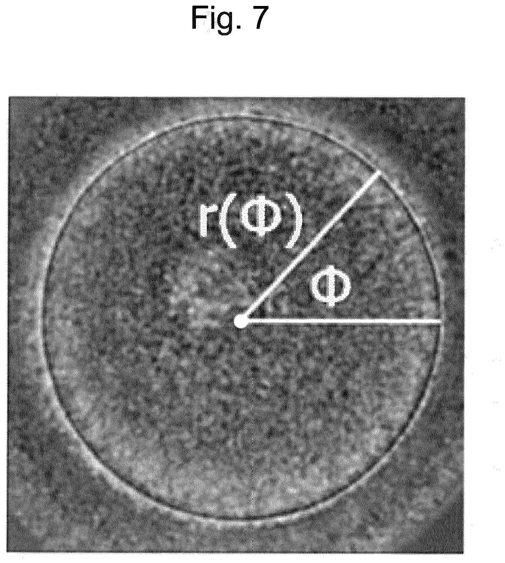

The contour extractor 31 similarly extracts the contour of the ovum for the images of all the frames constituting the moving image over the predetermined time. Next, the radius calculator 32 obtains a radius (strictly speaking, a distance from the center point to the contour) in each of directions at predetermined angular intervals $\Delta\theta$ around the center point of the ovum for each frame image (step S22). That is, as illustrated in Fig. 7, a radius $r(\varphi)$ is calculated for a plurality of predetermined angles $\varphi$. Here, as an example, the radius $r(\varphi)$ ($\varphi$ = 0 to 360°) in 360 directions is obtained for each frame image with $\Delta\theta = 1°$. The center point at the time of calculating the radius may be a gravity center position calculated from the contour.

The shape of the ovum, which is one type of cell, temporally changes due to fluctuation. The radial displacement amount calculator 33 calculates a radial displacement amount reflecting the shape fluctuation of the ovum on the basis of the information of an enormous number of radii for each frame image (step S23). For this purpose, the method disclosed in Non Patent Literature 4 is used here. Specifically, the radial displacement amount calculator 33 calculates a mean square amplitude (MSA) that is a function of a wavenumber q by performing a Fourier transform operation using the following Formula (1).

[Math. 1]

$$\langle u(q)^2 \rangle = \left\langle \left| \frac{2}{N} \sum_{n=0}^{N-1} \{r(n\Delta\theta) - \langle r(n\Delta\theta) \rangle\} e^{2\pi i q n / N} \right|^2 \right\rangle \quad \cdots (1)$$

In Formula (1), < > represents a temporal mean through all the frames. In addition, N is the number of data (samples) in angular directions per image, that is, $N = 360/\Delta\theta$.

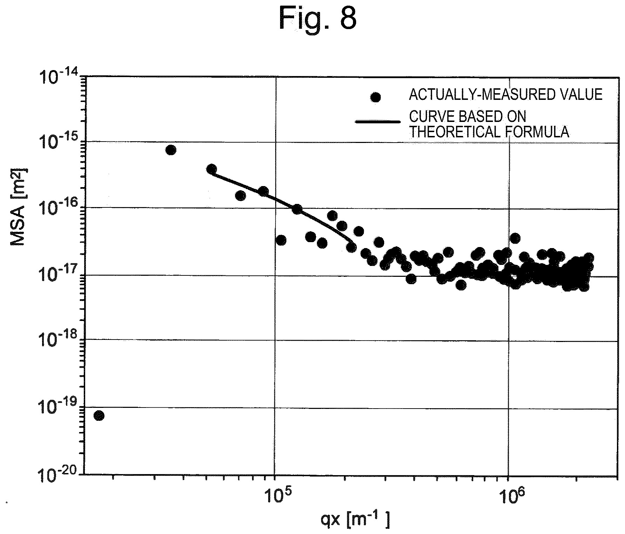

Formula (1) can be considered that a difference between the radius and the temporal mean of the radii is obtained for each direction, and a temporal change of a value obtained by adding the differences in all the directions is regarded as the function of the wavenumber by Fourier transform. In Fig. 8, the mean square amplitude values calculated by the above procedure on the basis of actual measurement data are plotted.

On the other hand, as disclosed in Non Patent Literature 4 as well, the following Formula (2) is known as a theoretical formula representing the elasticity of a cell.

[Math. 2]

$$\langle u(q_x)^2 \rangle = \frac{k_B T}{L} \sqrt{\frac{\kappa}{2(\sigma^2 - 4\kappa\gamma)}} \left( \frac{1}{\sqrt{2\kappa q_x^2 + \sigma - \sqrt{\sigma^2 - 4\kappa\gamma}}} \right.$$
$$\left. - \frac{1}{\sqrt{2\kappa q_x^2 + \sigma + \sqrt{\sigma^2 - 4\kappa\gamma}}} \right) \quad \cdots (2)$$

In Formula (2), $q_x$ is a continuous wavenumber corresponding to experimental q. In addition, L is the length in one dimensional direction of a cell (here, an ovum). In addition, $k_B$ is a Boltzmann constant, and T is an absolute temperature at the time of an experiment. Other parameters $\gamma$, $\sigma$, and $\kappa$ are unknown mechanical parameters, where $\gamma$ is a spring constant, $\sigma$ is surface tension, and $\kappa$ is bending elasticity. The mechanical parameter calculator 34 calculates the three unknown mechanical parameters by fitting Formula (2) to the mean square amplitudes calculated from the actual measurement data as described above (step S24). In Fig. 8, a curve in performing the fitting is indicated by a solid line. These three mechanical parameters are index values reflecting the mechanical properties of the ovum, that is, hardness and softness. That is, the mechanical properties of the ovum are quantified in this manner.

Next, the first evaluator 35 obtains information on the quality of the ovum from the mechanical parameter values of the ovum calculated as described above (step S25). In the ovum evaluation apparatus of the present embodiment, a machine learning method is used to evaluate the quality. That is, the first evaluator 35 determines whether the quality of the ovum is good or not by performing classification using a learned model based on a predetermined machine learning algorithm, in which the above three mechanical parameter values are input, and an evaluation result is output in which an ovum with good quality is "1" and an ovum with poor quality is "0".

The learned model is created in advance (for example, at a stage before shipment of the apparatus by a manufacturer) as follows.

That is, for one or more ova retrieved from each of a large number of patients, the mechanical parameters of each ovum are obtained according to the procedure as described above. On the other hand, a skilled embryologist evaluates the quality of each ovum during a process of performing normal insemination and subsequent culture of the ova, and leaves the evaluation result. The evaluation result by the embryologist may be binary information indicating whether the quality is good or not, such as the suitability of transfer and the suitability of cryopreservation. In addition, the evaluation result may also be multi-value information according to a known multi-grade evaluation method such as Veeck's classification and Gardner's classification. Of course, during a normal insemination and culture process, an obviously defective ovum may be discarded in a midway stage. In this case, the evaluation result indicating that the quality is poor can be obtained without decision by the embryologist.

In this way, it is possible to create a learned model, that is, an ovum quality discriminator by preparing a large number of training data sets each including actually-measured mechanical parameters (that is, input data) and an evaluation result (that is, correct answer data) for a large number of ova, and causing a neural network or the like to learn the training data. The learned model storage 350 stores this learned model.

The work of creating the learned model on the basis of the training data as described above may be performed by a user, but is usually performed by a manufacturer of the present apparatus or a manufacturer providing software.

When evaluating an unknown ovum, the first evaluator 35 receives mechanical parameter values obtained for the unknown ovum as input to the learned model, to be able to output a result indicating whether the quality of the ovum is good or not. This evaluation result is output from the display unit 6 through the main control unit 4. In a case where it is desired to simply obtain binary information such as the suitability of transfer, for example, as the evaluation result of the quality of the ovum, the first evaluator 35 may use a machine learning algorithm for binary classification. In addition, even when the correct answer data is binary data, it is possible to calculate a probability of the quality being good as a numerical value by using a machine learning algorithm for regression analysis such as logistic regression, for example. Therefore, in this case, for example, by comparing the calculated probability with a threshold value, it is possible to determine whether the quality of the ovum is good or not.

In addition, for example, by creating a learned model based on a machine learning algorithm for regression analysis using an objective numerical value such as a fertilization rate and an implantation rate as correct answer data, the first evaluator 35 can obtain the evaluation result of the quality of the ovum as the numerical value such as the fertilization rate and the implantation rate. Also in this case, the first evaluator 35 determines whether or not the numerical value of the probability output as the evaluation result is a predetermined threshold value or more, so that the result indicating whether the quality of the ovum is good or not can be obtained and displayed on the display unit 6. Of course, such a numerical value of the probability may be displayed together with the quality determination result, or only the numerical value of the probability may be displayed.

In addition, in infertility treatment, a plurality of ova are usually retrieved from a patient, and finally, it is necessary to narrow down the ova to one of them. Therefore, in a case where the probability that the quality of the ovum is good is obtained as the numerical value as described above, the first evaluator 35 can rank a plurality of ova retrieved from one patient on the basis of the numerical value and display the result of ranking on the display unit 6. The embryologist (doctor) who has confirmed this result can choose an ovum to be subjected to intracytoplasmic sperm injection from the plurality of ova or can select an appropriate ovum to be returned to the patient's body with reference to the ranking.

In addition, as a method for evaluating the quality of a fertilized ovum by an embryologist, a multi-grade evaluation method such as the Veeck's classification and the Gardner's classification described above are well known. The Veeck's classification is an index for evaluating the quality of an early embryo on Day 2 to Day 4 of culture in an ovum after insemination, and is classification with five grades. On the other hand, the Gardner's classification is an index for evaluating the state of a blastocyst on Day 5 to Day 6 of culture, and is classification with six grades. These are methods for evaluating an ovum after insemination. A learned model may also be created by performing learning using training data in which a result of grading in the Veeck's classification or the Gardner's classification is used as correct answer data, and the result of grading in the Veeck's classification or the Gardner's classification may be obtained from mechanical parameter values for an ovum before insemination using this learned model.

Here, it is a matter of course that various known algorithms that can be used for classification and regression as machine learning, for example, logistic regression, support vector machine, k-nearest neighbor algorithm, random forest, linear regression, regularization, neural network, and the like can be appropriately used.

Furthermore, when the quality of the ovum is evaluated, not only the mechanical parameters of the ovum may be used as an evaluation index, but also various information other than the mechanical parameters related to the ovum, for example, other information such as the size of the ovum may be added as the evaluation index. In addition, not the ovum itself but information specific to a patient, for example, information such as the age, past results of artificial insemination, and medical history of the patient may be added as the evaluation index.

In particular, it is known that the age of a patient greatly affects the quality of an ovum. Thus, it is highly appropriate to use the information of the age of the patient as one of evaluation indexes. In this case, the age may be treated as the input of the learned model equivalent to the mechanical parameters, but instead, a result obtained by machine learning, for example, a threshold value on up to what rank is determined to be good in quality in the result of ranking a plurality of ova, a threshold value on what percentage or more is determined to be good in quality with regard to the probability of the quality being good, and the like may be changed according to the age of the patient.

The present inventors have experimentally verified whether or not an ovum retrieved from an actual patient can be accurately evaluated using the learned model created so as to evaluate the quality of an ovum from the viewpoint of the suitability of transfer into a mother's body. In this verification, quality evaluation based on actually-measured mechanical parameters has been performed for a plurality of ova retrieved per patient from 14 patients. As a result, it has been possible to confirm that the result of the evaluation using the learned model is almost equivalent to the evaluation of the quality of the ovum by a skilled embryologist.

When the evaluation of the quality of the ovum before insemination is completed as described above, the embryologist refers to the evaluation result, selects one or more good-quality ova from a plurality of ova retrieved from one patient, and discards the others. Then, the embryologist performs an operation of intracytoplasmic sperm injection on the selected good-quality ovum (ova).

The method of intracytoplasmic sperm injection is exactly the same as the conventional method, in which the embryologist injects sperm by inserting an injection pipette into the ovum while observing the ovum with a microscope. Here, this intracytoplasmic sperm injection work is performed under observation by the microscopic observation unit 1 of the ovum evaluation apparatus illustrated in Fig. 3. The image acquiring unit 10 photographs the state of the ovum during the work, particularly the state of the ovum from a time point immediately before the injection pipette is inserted into the ovum until a predetermined time elapses after the injection pipette is removed. The obtained moving image data is transferred to the data processing unit 3 and temporarily stored in the image data storage 30.

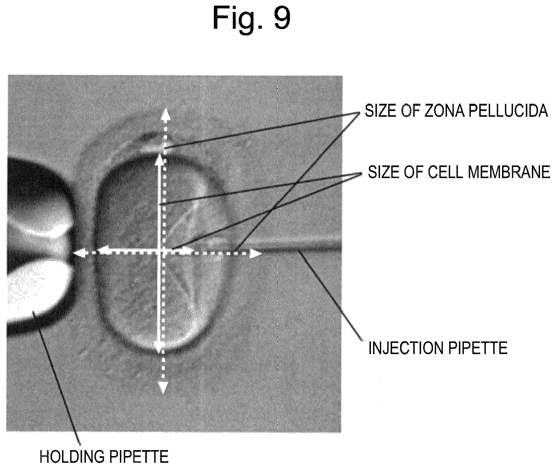

After that, the embryologist (or another person in charge) performs a predetermined operation on the input unit 5. In response to this operation, the data processing unit 3 performs the ovum evaluation processing during insemination as the second-stage ovum quality evaluation on the basis of the stored moving image data. This ovum evaluation will be described in detail with reference to Fig. 9. As described above, the operation of inserting the injection pipette into the ovum is an essential operation for intracytoplasmic sperm injection, and is not an operation involving contact with or invasion upon the ovum other than the normal operation. Thus, it can be said that this ovum evaluation is also non-invasive evaluation.

Fig. 9 is an image showing the state of the ovum when the injection pipette is inserted into the ovum for intracytoplasmic sperm injection.

As illustrated in Fig. 9, when sperm is injected into the ovum, the ovum is fixed by a holding pipette. The tip of the injection pipette is pierced through the zona pellucida of the fixed ovum and is further pierced through the cell membrane of the ovum to be pushed deep into the ooplasm. At about the same time, the sperm is injected into the ooplasm and then the injection pipette is quickly withdrawn from the ovum. When the tip of the injection pipette is pierced into the zona pellucida, the zona pellucida is pushed by the tip of the injection pipette to be once recessed inward. When the tip of the injection pipette is further advanced, a hole is opened in a part of the zona pellucida. That is, with the movement of the injection pipette, the zona pellucida is greatly deformed, and when a hole is opened in the zona pellucida, the deformation is relaxed and the zona pellucida returns to a substantially original state. The same applies to the cell membrane of the ovum, and with the movement of the injection pipette, the cell membrane is greatly deformed so as to be recessed inward, and when a hole is opened in the cell membrane, the deformation is relaxed and the cell membrane returns to a substantially original state.

In the data processing unit 3, the aspect ratio calculator 36 detects, from the stored moving image, an image in a state where the zona pellucida is deformed the most immediately before the deformation starts to be relaxed after the zona pellucida is greatly deformed at the time of insertion of the injection pipette as described above. The aspect ratio calculator 36 calculates the length in a minor axis direction and the length in a major axis direction of the zona pellucida from the image, and obtains the ratio of the lengths as an aspect ratio. The length in the minor axis direction is the width of the zona pellucida at a position passing through substantially the center of the ovum along an advancing and retracting direction (horizontal direction in Fig. 9) of the injection pipette. On the other hand, the length in the major axis direction is the width of the zona pellucida at a position passing through substantially the center of the ovum along a direction (vertical direction in Fig. 9) orthogonal to the advancing and retracting direction of the injection pipette.

The above aspect ratio of the zona pellucida, which is a part of the ovum, is affected by the mechanical properties of the zona pellucida, specifically, the elasticity. In other words, the above aspect ratio is a useful index for measuring the mechanical properties of the ovum, and the mechanical properties of the ovum affect the quality of the ovum. Thus, the quality of the ovum can be evaluated on the basis of the aspect ratio. Therefore, the second evaluator 37 receives the value of the above aspect ratio and compares the value with a predetermined threshold value to determine whether the quality of the ovum is good or not. Then, the determination result of the quality of the ovum is displayed on the display unit 6 through the main control unit 4. Of course, similarly to the first evaluator 35, the quality of the ovum may be evaluated together with an index other than the aspect ratio, for example, personal information such as the age of the patient.

In addition, the aspect ratio of not the zona pellucida but the cell membrane, that is, the aspect ratio in a state where the cell membrane is deformed the most immediately before the deformation starts to be relaxed after the cell membrane is greatly deformed at the time of insertion of the injection pipette, may be calculated, and the quality of the ovum may be determined using this aspect ratio. Furthermore, the quality of the ovum may be determined using both the aspect ratio of the zona pellucida and the aspect ratio of the cell membrane.

For example, even in a case where the quality is determined to be good in the ovum evaluation before insemination, the quality may be determined to be poor in the ovum evaluation during insemination. In this case, the fertilization rate or the implantation rate is possibly poor. Therefore, by excluding the fertilized ovum evaluated to be poor in quality during insemination from a culture target, the culture loss of the fertilized ovum can be reduced, and the incubator can be effectively used.

In the above description, the aspect ratio at the time of maximum deformation of the zona pellucida and/or the cell membrane is used as the ovum evaluation during insemination. However, the quality of the ovum can also be evaluated using other information as described below reflecting the mechanical properties of the cell membrane or the zona pellucida.

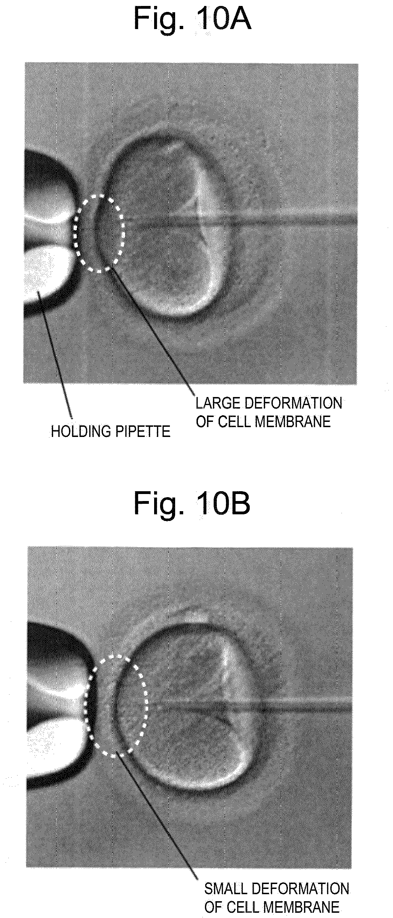

Figs. 10A and 10B are examples of images showing the state of the ovum fixed by the holding pipette. In the example of Fig. 10A, the cell membrane is deformed so as to protrude clearly by being sucked by the holding pipette. On the other hand, in the example of Fig. 10B, the cell membrane hardly protrudes although being sucked by the holding pipette. This difference in deformation reflects a difference in the elasticity of the ovum. Therefore, the second evaluator 37 can determine whether the quality of the ovum is good or not by determining the presence or absence of deformation by image processing, for example. Alternatively, the deformation amount (protrusion amount) may be quantified, and the quantitative value may be compared with a threshold value to determine whether the quality of the ovum is good or not.

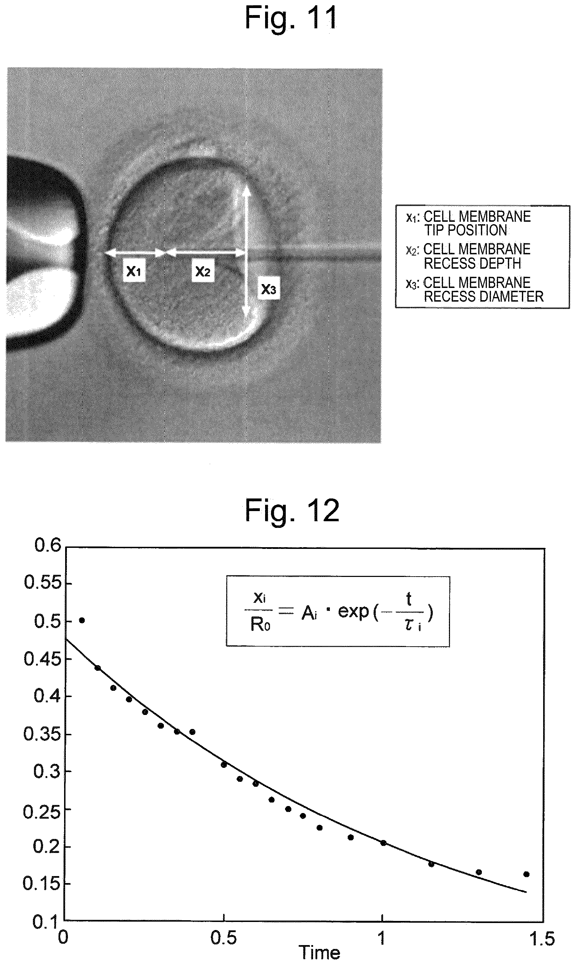

In addition, the second evaluator 37 can obtain, from the moving image acquired during the insemination work, a time required for the deformation of the cell membrane to be relaxed (return to the original state) (hereinafter, referred to as "relaxation time") from a moment when the injection pipette pierces the cell membrane, to determine whether the quality of the ovum is good or not on the basis of the relaxation time. Fig. 11 is a drawing illustrating a portion to observe a change in the cell membrane in obtaining an index value of deformation relaxation of the ovum after a time point of opening a hole by insertion of the injection pipette. Fig. 12 is a drawing illustrating an example of a temporal change in deformation of the cell membrane (deformation relaxation of the ovum) after being pierced by the injection pipette.

Fig. 11 is an image showing a state immediately before the tip of the injection pipette pierces the cell membrane. Here, attention is paid to three sizes $x_1$, $x_2$, and $x_3$ illustrated in the drawing as the deformation of the cell membrane, $x_1$ is a distance from the tip position of the injection pipette immediately before a hole is opened in the cell membrane to the position of the cell membrane facing the tip ahead of the tip. $x_2$ is the depth of a recess in the cell membrane formed by being pressed by the injection pipette. $x_3$ is the diameter of the rim of the recess in the cell membrane formed by being pressed by the injection pipette. All of these values indicate maximum values immediately before the tip of the injection pipette pierces the cell membrane, and decrease with time after the piercing.

Therefore, the data processing unit 3 calculates $x_1$, $x_2$, and $x_3$ after the time point when the tip of the injection pipette pierces the cell membrane in the frame images at predetermined time intervals included in the moving image. The actually-measured values change as plotted in Fig. 12, for example. Therefore, the second evaluator 37 calculates a relaxation time $\tau_i$ by fitting Formula (3) which is a theoretical formula to the actually-measured values:

$$(x_i/R_0) = A_i \cdot \exp(-t/\tau_i) \quad \dots (3)$$

where i = 1 to 3. In addition, $R_0$ is the diameter of the ovum, and Ai is the deformation strength. The larger the elasticity, the shorter the relaxation time. Thus, three relaxation times $\tau_i$ can be used as the evaluation index, and these can be appropriately combined to evaluate the quality of the ovum. Of course, the evaluation result of the ovum may be derived by inputting the three relaxation times $\tau_i$ using the above-described machine learning method.

In addition, the quality of the ovum may be evaluated using the relaxation time of at least one size of $x_1$, $x_2$, and $x_3$ illustrated in Fig. 11. In addition, the quality of the ovum may be evaluated using at least one size of $x_1$, $x_2$, and $x_3$ at the time point when the cell membrane is deformed the most as the evaluation index.

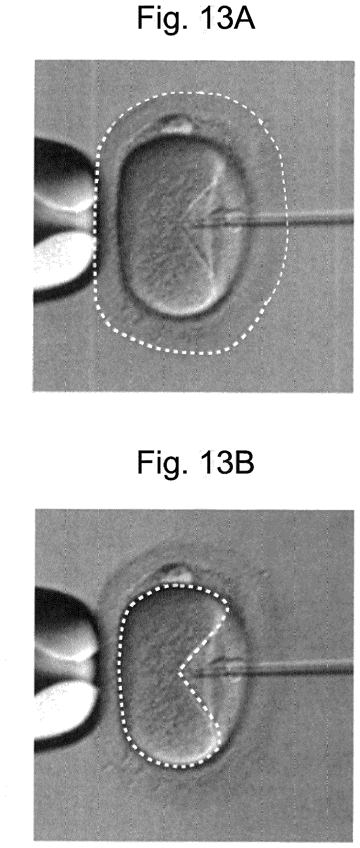

Furthermore, instead of the size (length) of a certain portion, the area of a portion surrounded by the zona pellucida or the area of a portion surrounded by the cell membrane can be used. Fig. 13A is a drawing illustrating a portion surrounded by the zona pellucida and Fig. 13B is a drawing illustrating a portion surrounded by the cell membrane in the image of the ovum. A change in the area of each of the above portions when the zona pellucida or the cell membrane is deformed the most immediately before the tip of the injection pipette pierces the cell membrane or the area of each of the above portions during deformation relaxation after the moment when the tip of the injection pipette pierces the cell membrane also reflects the elasticity of the ovum. Therefore, it is also possible to evaluate the quality of the ovum by using such information as the evaluation index.

Of course, it is also possible to improve the accuracy and reliability of the evaluation of the quality of the ovum by using the plurality of evaluation indexes described above in combination or using the evaluation results obtained from the respective evaluation indexes in combination.

In addition, since the evaluation of the quality of the ovum by the first evaluator 35 and the evaluation of the quality of the ovum by the second evaluator 37 are completely independent of each other, it is obvious that only one of them may be performed. Of course, it is obviously very convenient to use both in intracytoplasmic sperm injection because it is possible to narrow down the ova to be fertilized and to narrow down the ova (fertilized ova) to be cultured. In addition, it goes without saying that the evaluation of the quality of the ovum by the first evaluator 35 is also useful in choosing the ovum to be subjected to in vitro fertilization instead of intracytoplasmic sperm injection.

In addition, in the ovum evaluation apparatus of the above-described embodiment, both the ovum evaluation before insemination and the ovum evaluation during insemination can be performed. However, these can be performed in separate apparatuses. In addition, in the above description, the ovum evaluation apparatus outputs the evaluation result or the determination result of the quality of the set ovum, and the embryologist selects an ovum with reference to the evaluation result or the determination result. However, it is also possible to incorporate a function of choosing an ovum on the basis of the evaluation result of the quality of the ovum into the ovum evaluation apparatus. At this time, only the ovum determined to be the best in quality may be selected, or conversely, only the ovum estimated to be obviously defective may be automatically discarded and the others may be left. That is, the final selection/choosing of the ovum may be decided by an embryologist, or the selection/choosing of the ovum may be automatically performed without such decision.

Furthermore, the above-described embodiment and the above-described various modifications are merely examples of the present invention, and it is a matter of course that modifications, changes, additions, and the like appropriately made within the scope of the gist of the present invention are included in the claims of the present application.

[Various Modes]

A person skilled in the art can understand that the previously described illustrative embodiment is a specific example of the following modes of the present invention.

(First Mode) One mode of an ovum evaluation method according to the present invention is a method for evaluating an ovum, the method including:

an analysis step of analyzing a deformation state of a target ovum using an image obtained by photographing the target ovum to non-invasively obtain an index value obtained by quantifying a mechanical property of the target ovum; and
an evaluation step of evaluating a quality of the target ovum on the basis of the index value obtained in the analysis step.

One mode of an ovum evaluation apparatus according to the present invention includes:

an analyzer configured to receive an image obtained by photographing a target ovum, and analyze a deformation state of the target ovum using the image to non-invasively obtain an index value obtained by quantifying a mechanical property of the target ovum; and
an evaluator configured to evaluate a quality of the target ovum on the basis of the index value obtained by the analyzer.

One mode of an ovum evaluation program according to the present invention is a program for evaluating an ovum using a computer, the program causing a computer to execute:

an analysis step of analyzing a deformation state of a target ovum using an image obtained by photographing the target ovum to non-invasively obtain an index value obtained by quantifying a mechanical property of the target ovum; and
an evaluation step of evaluating a quality of the target ovum on the basis of the index value obtained in the analysis step.

According to the ovum evaluation method, the ovum evaluation apparatus, and the ovum evaluation program of the above first mode, the quality of the target ovum can be non-invasively evaluated accurately on the basis of the index value obtained by quantifying the mechanical property, that is, with theoretical grounding. As a result, the ovum is not damaged during evaluation of the quality of the ovum. For example, the ovum after the evaluation can be favorably used for the original purpose. In addition, the workload of an embryologist is reduced, and a variation in quality evaluation depending on the skill of an embryologist in charge is eliminated, to improve the reliability of the quality evaluation of the ovum. Furthermore, data in evaluating the quality of the ovum remain as a numerical value, which makes it easy for an embryologist or the like to verify whether or not the quality evaluation is appropriate later.

(Second Mode) In an ovum evaluation method according to the first mode, the target ovum may be an ovum before insemination, the image may be a moving image or time-lapse images of the target ovum, and

the analysis step may include: a contour extraction step of extracting a contour of the target ovum from each of a plurality of images constituting the moving image or the time-lapse images; a displacement amount acquisition step of obtaining a displacement amount of the contour of the target ovum; and a mechanical parameter calculation step of calculating, as the index value, a mechanical parameter of the target ovum from the displacement amount of the contour of the ovum.

Similarly, in an ovum evaluation apparatus according to the first mode, the target ovum may be an ovum before insemination, the image may be a moving image or time-lapse images of the target ovum, and

the analyzer may include: a contour extraction processor configured to extract a contour of the target ovum from each of a plurality of images constituting the moving image or the time-lapse images; a displacement amount acquisition processor configured to obtain a displacement amount of the contour of the target ovum; and a mechanical parameter calculation processor configured to calculate, as the index value, a mechanical parameter of the target ovum from the displacement amount of the contour of the ovum.

The shape of the ovum, which is one type of cell, temporally changes due to fluctuation. The mode of the shape change of the ovum due to fluctuation is affected by elasticity reflecting the hardness or the like of the ovum. For example, in the above ovum evaluation method, the change in the shape of the ovum is obtained as the information of the displacement amount of the contour in the contour extraction step and the displacement amount acquisition step. In the mechanical parameter calculation step, the mechanical parameter of the ovum is calculated from the information of the displacement amount of the contour. As a result, according to the ovum evaluation method and the ovum evaluation apparatus of the second mode, the quality of the target ovum can be accurately evaluated on the basis of the mechanical parameter of the ovum.

(Third Mode) In an ovum evaluation method according to the second mode, in the mechanical parameter calculation step, the mechanical parameter may be obtained by performing parameter fitting using a theoretical formula representing elasticity of a cell.

Similarly, in an ovum evaluation apparatus according to the second mode, the mechanical parameter calculation processor may be configured to obtain the mechanical parameter by performing parameter fitting using a theoretical formula representing elasticity of a cell, on an actually-measured value representing the displacement amount of the contour of the target ovum.

In general, in order to grasp mechanical properties such as the elasticity of an object, some force is applied to the object to measure a deformation rate or the like of the object with respect to the force. On the other hand, according to the ovum evaluation method and the ovum evaluation apparatus of the third mode, information on the elasticity of the ovum can be accurately obtained without even indirectly applying any force to the ovum or regulating the movement of the ovum. In addition, in order to observe the shape change due to fluctuation, it is only required to photograph the appearance for a time of about 1 minute at the longest, usually shorter than that, and it is possible to almost avoid substantial damage to the ovum.

(Fourth Mode) In an ovum evaluation method according to any one of the first to third modes, in the evaluation step, the quality of the ovum may be evaluated using a discriminator obtained by machine learning using training data including an evaluation result of an ovum by an embryologist in at least any of stages from fertilization to implantation or training data in which information of a probability of success or failure in any of the stages is known.

Similarly, in an ovum evaluation apparatus according to any one of the first to third modes, the evaluator may be configured to evaluate the quality of the ovum using a discriminator created in advance by machine learning using training data including an evaluation result of an ovum by an embryologist in at least any of stages from fertilization to implantation or training data in which information of a probability of success or failure in any of the stages is known.

Any machine learning algorithm may be used. According to the ovum evaluation method and the ovum evaluation apparatus of the fourth mode, it is possible to accurately evaluate an ovum before culture by reflecting a decision result of the suitability of transfer of a fertilized ovum or the like by an expert embryologist having a high level of skill. In addition, according to the ovum evaluation method and the ovum evaluation apparatus of the fourth mode, the quality of the ovum can be accurately evaluated by reflecting past success/failure results such as a fertilization rate and an implantation rate.

(Fifth Mode) In an ovum evaluation method according to any one of the first to fourth modes, in the evaluation step, the quality of the ovum may be evaluated using patient-specific information including an age in addition to the index value.

Similarly, in an ovum evaluation apparatus according to any one of the first to fourth modes, the evaluator may be configured to evaluate the quality of the ovum using patient-specific information including an age that is input in advance in addition to the index value.

It is well known that the fertilization rate and the implantation rate decrease as the age of a patient increases. In many cases, the influence of the increase in the age of a patient appears in a decrease in the elasticity of an ovum, but other factors that have not yet been sufficiently elucidated are also conceivable. To address the problem, according to the ovum evaluation method and the ovum evaluation apparatus of the fifth mode, the patient-specific information such as an age is additionally considered in the evaluation, which makes it possible to more accurately evaluate the quality of the ovum and increase the possibility of pregnancy.

(Sixth Mode) In an ovum evaluation method according to any one of the first to fifth modes, in the evaluation step, information on the quality of the ovum may be acquired for each of a plurality of ova retrieved from one patient, and the plurality of ova may be ranked using the information.

Similarly, in an ovum evaluation apparatus according to any one of the first to fifth modes, the evaluator may be configured to rank a plurality of ova using information on the quality of the ovum obtained for each of the plurality of ova.

According to the ovum evaluation method and the ovum evaluation apparatus of the sixth mode, an operation of intracytoplasmic sperm injection can be performed on one ovum estimated to have the highest probability of pregnancy or a small number of ova estimated to have a relatively high possibility of pregnancy among the plurality of ova retrieved from the patient. In addition, in a case where there are a plurality of fertilized ova, one estimated to have the highest probability of pregnancy can be chosen and transferred, and the rest can be cryopreserved.

(Seventh Mode) In an ovum evaluation method according to the first mode, the image may be an image photographed when a needle is inserted into the target ovum for intracytoplasmic sperm injection, and in the analysis step, an index value reflecting a degree of deformation in maximum deformation of a zona pellucida or a cell membrane associated with the insertion of the needle or an index value reflecting a degree of relaxation of deformation of the zona pellucida or the cell membrane associated with the insertion of the needle may be obtained.

Similarly, in an ovum evaluation apparatus according to the first mode, the image may be an image photographed when a needle is inserted into the target ovum for intracytoplasmic sperm injection, and the analyzer may be configured to obtain an index value reflecting a degree of deformation in maximum deformation of a zona pellucida or a cell membrane associated with the insertion of the needle or an index value reflecting a degree of relaxation of deformation of the zona pellucida or the cell membrane associated with the insertion of the needle.

In the ovum evaluation method and the ovum evaluation apparatus of the seventh mode, the quality of the ovum is evaluated from the index value calculated on the basis of the image photographed when the intracytoplasmic sperm injection is performed. As described above, it can be said that the evaluation of the quality of the ovum is performed non-invasively in that special invasive measurement or observation for evaluating the quality of the ovum is not performed.

According to the ovum evaluation method and the ovum evaluation apparatus of the seventh mode, quantitative evaluation using a specific numerical value can be performed instead of sensory and qualitative evaluation such as being elastic or being poorly elastic, which has been conventionally performed by an embryologist at the time of performing intracytoplasmic sperm injection. This improves the accuracy and reliability of the evaluation of the quality of the ovum. Furthermore, data in evaluating the quality of the ovum remain as a numerical value, and the accuracy of the evaluation or the like can be easily verified.

(Eighth Mode) In an ovum evaluation method according to the seventh mode, the index value reflecting the degree of deformation in the maximum deformation may be a ratio of widths in two directions of a needle insertion direction and a direction orthogonal to the needle insertion direction in the maximum deformation of the zona pellucida or the cell membrane of the target ovum.

Similarly, in an ovum evaluation apparatus according to the seventh mode, the index value reflecting the degree of deformation in the maximum deformation may be a ratio of widths in two directions of a needle insertion direction and a direction orthogonal to the needle insertion direction in the maximum deformation of the zona pellucida or the cell membrane of the target ovum.

According to the ovum evaluation method and the ovum evaluation apparatus of the eighth mode, the quality of the ovum can be accurately evaluated on the basis of the index value accurately reflecting the degree of elasticity of the ovum at the time of intracytoplasmic sperm injection.

(Ninth Mode) An ovum evaluation method according to any one of the first to eighth modes may further include a choosing step of choosing an ovum using an evaluation result in the evaluation step.

According to the ovum evaluation method of the ninth mode, the load of an ovum choosing work by an embryologist is reduced. In addition, the ovum can be chosen on the basis of the evaluation result having theoretical grounding rather than sensory evaluation, and the reliability of choosing is improved.

(Tenth Mode) In an ovum evaluation method according to any one of the first to sixth modes, the quality of the ovum which is an ovum before insemination may be evaluated in the analysis step and the evaluation step, the method further including:

a second analysis step of analyzing a deformation mode of an ovum chosen according to a result of the evaluation using an image photographed when a needle is inserted into the ovum for intracytoplasmic sperm injection to non-invasively obtain an index value obtained by quantifying a mechanical property of the ovum; and
a second evaluation step of evaluating a quality of the ovum subjected to the intracytoplasmic sperm injection on the basis of the index value obtained in the second analysis step.

Similarly, an ovum evaluation apparatus according to any one of the first to sixth modes may further include:

a second analyzer configured to analyze a deformation mode of an ovum chosen according to a result of evaluating the quality of the ovum which is an ovum before insemination by the analyzer and the evaluator, using an image photographed when a needle is inserted into the ovum for intracytoplasmic sperm injection, to non-invasively obtain an index value obtained by quantifying a mechanical property of the ovum; and
a second evaluator configured to evaluate a quality of the ovum subjected to the intracytoplasmic sperm injection on the basis of the index value obtained by the second analyzer.

In the ovum evaluation method and the ovum evaluation apparatus of the tenth mode, specifically, when the quality of the ovum is evaluated by, for example, the ovum evaluation method of the second mode, the ovum is chosen on the basis of the result, and then intracytoplasmic sperm injection is performed on the chosen ovum, the quality of the ovum can be evaluated again by the ovum evaluation method of the seventh mode. As described above, by evaluating the quality of the ovum in two stages of before insemination and during insemination, the accuracy and reliability of the evaluation can be improved, and the possibility of pregnancy can be increased. In addition, it is possible to reduce a culture loss and a cryopreservation loss of the fertilized ovum and to reduce the cost of assisted reproductive technology.

REFERENCE SIGNS LIST

1... Microscopic Observation Unit
10... Image Acquiring Unit
100... Ovum
2... Photographing Control Unit
3... Data Processing Unit
30... Image Data Storage
31... Contour Extractor
32... Radius Calculator
33... Radial Displacement Amount Calculator
34... Mechanical Parameter Calculator
35... First Evaluator
350... Model Storage
36... Aspect Ratio Calculator
37... Second Evaluator
4... Main Control Unit
5... Input Unit
6... Display Unit

**Claims**

1. An ovum evaluation method for evaluating an ovum, the method comprising:

an analysis step of analyzing a deformation state of a target ovum using an image obtained by photographing the target ovum to non-invasively obtain an index value obtained by quantifying a mechanical property of the target ovum; and
an evaluation step of evaluating a quality of the target ovum on a basis of the index value obtained in the analysis step.

2. The ovum evaluation method according to claim 1, wherein

the target ovum is an ovum before insemination, the image is a moving image or time-lapse images of the target ovum, and

the analysis step includes: a contour extraction step of extracting a contour of the target ovum from each of a plurality of images constituting the moving image or the time-lapse images; a displacement amount acquisition step of obtaining a displacement amount of the contour of the target ovum; and a mechanical parameter calculation step of calculating, as the index value, a mechanical parameter of the target ovum from the displacement amount of the contour of the ovum.

3. The ovum evaluation method according to claim 2, wherein in the mechanical parameter calculation step, the mechanical parameter is obtained by performing parameter fitting using a theoretical formula representing elasticity of a cell, on an actually-measured value representing the displacement amount of the contour of the target ovum.

4. The ovum evaluation method according to claim 1, wherein in the evaluation step, the quality of the ovum is evaluated using a discriminator obtained by machine learning using training data including an evaluation result of an ovum by an embryologist in at least any of stages from fertilization to implantation or training data in which information of a probability of success or failure in any of the stages is known.

5. The ovum evaluation method according to claim 1, wherein in the evaluation step, the quality of the ovum is evaluated using patient-specific information including an age in addition to the index value.

6. The ovum evaluation method according to claim 1, wherein in the evaluation step, information on the quality of the ovum is acquired for each of a plurality of ova retrieved from one patient, and the plurality of ova are ranked using the information.

7. The ovum evaluation method according to claim 1, wherein

the image is an image photographed when a needle is inserted into the target ovum for intracytoplasmic sperm injection, and

in the analysis step, an index value reflecting a degree of deformation in maximum deformation of a zona pellucida or a cell membrane associated with the insertion of the needle or an index value reflecting a degree of relaxation of deformation of the zona pellucida or the cell membrane associated with the insertion of the needle is obtained.

8. The ovum evaluation method according to claim 7, wherein the index value reflecting the degree of deformation in the maximum deformation is a ratio of widths in two directions of a needle insertion direction and a direction orthogonal to the needle insertion direction in the maximum deformation of the zona pellucida or the cell membrane of the target ovum.

9. The ovum evaluation method according to claim 1, further comprising a choosing step of choosing an ovum using an evaluation result in the evaluation step.

10. The ovum evaluation method according to claim 1, wherein

the quality of the ovum which is an ovum before insemination is evaluated in the analysis step and the evaluation step, the method further comprising:

a second analysis step of analyzing a deformation mode of an ovum chosen according to a result of the evaluation using an image photographed when a needle is inserted into the ovum for intracytoplasmic sperm injection to non-invasively obtain an index value obtained by quantifying a mechanical property of the ovum; and

a second evaluation step of evaluating a quality of the ovum subjected to the intracytoplasmic sperm injection on a basis of the index value obtained in the second analysis step.

11. An ovum evaluation apparatus comprising:

an analyzer configured to receive an image obtained by photographing a target ovum, and analyze a deformation state of the target ovum using the image to non-invasively obtain an index value obtained by quantifying a mechanical property of the target ovum; and

an evaluator configured to evaluate a quality of the target ovum on a basis of the index value obtained by the analyzer.

12. The ovum evaluation apparatus according to claim 11, wherein

the target ovum is an ovum before insemination, the image is a moving image or time-lapse images of the target ovum, and

the analyzer includes: a contour extraction processor configured to extract a contour of the target ovum from each of a plurality of images constituting the moving image or the time-lapse images; a displacement amount acquisition processor configured to obtain a displacement amount of the contour of the target ovum; and a mechanical parameter calculation processor configured to calculate, as the index value, a mechanical parameter of the target ovum from the displacement amount of the contour of the ovum.

13. The ovum evaluation apparatus according to claim 12, wherein the mechanical parameter calculation processor is configured to obtain the mechanical parameter by performing parameter fitting using a theoretical formula representing elasticity of a cell, on an actually-measured value representing the displacement amount of the contour of the target ovum.

14. The ovum evaluation apparatus according to claim 11, wherein the evaluator is configured to evaluate the quality of the ovum using a discriminator created in advance by machine learning using training data including an evaluation result of an ovum by an embryologist in at least any of stages from fertilization to implantation or training data in which information of a probability of success or failure in any of the stages is known.

15. The ovum evaluation apparatus according to claim 11, wherein the evaluator is configured to evaluate the quality of the ovum using patient-specific information including an age that is input in advance in addition to the index value.

16. The ovum evaluation apparatus according to claim 11, wherein the evaluator is configured to rank a plurality of ova using information on the quality of the ovum obtained for each of the plurality of ova.

17. The ovum evaluation apparatus according to claim 11, wherein

the image is an image photographed when a needle is inserted into the target ovum for intracytoplasmic sperm injection, and

the analyzer is configured to obtain an index value reflecting a degree of deformation in maximum deformation of a zona pellucida or a cell membrane associated with the insertion of the needle or an index value reflecting a degree of relaxation of deformation of the zona pellucida or the cell membrane associated with the insertion of the needle.

18. The ovum evaluation apparatus according to claim 17, wherein the index value reflecting the degree of deformation in the maximum deformation is a ratio of widths in two directions of a needle insertion direction and a direction orthogonal to the needle insertion direction in the maximum deformation of the zona pellucida or the cell membrane of the target ovum.

19. The ovum evaluation apparatus according to claim 11, further comprising:

a second analyzer configured to analyze a deformation mode of an ovum chosen according to a result of evaluating the quality of the ovum which is an ovum before insemination by the analyzer and the evaluator, using an image photographed when a needle is inserted into the ovum for intracytoplasmic sperm injection, to non-invasively obtain an index value obtained by quantifying a mechanical property of the ovum; and

a second evaluator configured to evaluate a quality of the ovum subjected to the intracytoplasmic sperm injection on a basis of the index value obtained by the second analyzer.

20. An ovum evaluation program for evaluating an ovum using a computer, the program causing a computer to execute:

an analysis step of analyzing a deformation state of a target ovum using an image obtained by photographing the target ovum to non-invasively obtain an index value obtained by quantifying a mechanical property of the target ovum; and
an evaluation step of evaluating a quality of the target ovum on a basis of the index value obtained in the analysis step.

# Fig. 1

START

S1 — RETRIEVE OVUM

S2 — OVUM EVALUATION PROCESSING BEFORE INSEMINATION

S3 — SELECT OVUM

S4 — INTRACYTOPLASMIC SPERM INJECTION OPERATION

S5 — OVUM EVALUATION PROCESSING DURING INSEMINATION

S6 — SELECT OVUM (FERTILIZED OVUM)

S7 — CULTURE/TRANSFER

# Fig. 2

OVUM EVALUATION PROCESSING
BEFORE INSEMINATION

S20 | PHOTOGRAPH TARGET OVUM OVER PREDETERMINED TIME

S21 | EXTRACT CONTOUR OF OVUM IN EACH FRAME IMAGE CONSTITUTING ACQUIRED MOVING IMAGE

S22 | CALCULATE RADIUS FROM CONTOUR OF OVUM FOR EACH FRAME IMAGE

S23 | CALCULATE RADIUS CHANGE AMOUNT OF OVUM

S24 | CALCULATE THREE TYPES OF MECHANICAL PARAMETERS BY FITTING RADIUS CHANGE AMOUNT OF OVUM TO THEORETICAL FORMULA

S25 | DERIVE INFORMATION ON QUALITY OF OVUM USING LEARNED MODEL IN WHICH THREE TYPES OF MECHANICAL PARAMETERS ARE USED AS EVALUATION INDEX

END

# Fig. 3

PHOTOGRAPHING CONTROL UNIT — 2

1

10

100

DATA PROCESSING UNIT — 3

IMAGE DATA STORAGE — 30

CONTOUR EXTRACTOR — 31

RADIUS CALCULATOR — 32

RADIAL DISPLACEMENT AMOUNT CALCULATOR — 33

MECHANICAL PARAMETER CALCULATOR — 34

FIRST EVALUATOR — 35

LEARNED MODEL STORAGE — 350

ASPECT RATIO CALCULATOR — 36

SECOND EVALUATOR — 37

MAIN CONTROL UNIT — 4

INPUT UNIT — 5

DISPLAY UNIT — 6

# Fig. 4

CELL
MEMBRANE

ZONA
PELLUCIDA

U

# Fig. 5

Fig. 6A

$$[x_i] = \frac{x_{i-2} + x_{i-1} + x_i + x_{i+1} + x_{i+2}}{5}$$

Fig. 6B

Fig. 6C

# Fig. 7

# Fig. 8

# Fig. 9

SIZE OF ZONA PELLUCIDA

SIZE OF CELL MEMBRANE

INJECTION PIPETTE

HOLDING PIPETTE

# Fig. 10A

LARGE DEFORMATION
OF CELL MEMBRANE

HOLDING PIPETTE

# Fig. 10B

SMALL DEFORMATION
OF CELL MEMBRANE

# Fig. 11

x₁: CELL MEMBRANE
TIP POSITION

x₂: CELL MEMBRANE
RECESS DEPTH

x₃: CELL MEMBRANE
RECESS DIAMETER

# Fig. 12

$$\frac{x_i}{R_0} = A_i \cdot \exp\left(-\frac{t}{\tau_i}\right)$$

## Fig. 13A

## Fig. 13B

## INTERNATIONAL SEARCH REPORT

| | International application No. |
|---|---|
| | **PCT/JP2022/036466** |

**A. CLASSIFICATION OF SUBJECT MATTER**

*C12Q 1/06*(2006.01)i; *C12M 1/34*(2006.01)i
FI:    C12Q1/06; C12M1/34 A

According to International Patent Classification (IPC) or to both national classification and IPC

**B. FIELDS SEARCHED**

Minimum documentation searched (classification system followed by classification symbols)

C12Q1/06; C12M1/34

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2012-531584 A (PHASE HOLOGRAPHIC IMAGING PHI AB) 10 December 2012 (2012-12-10) claims, paragraphs [0023], [0043], [0045], [0076], [0088] | 1, 2, 5-12, 15-20 |
| Y | claims, paragraphs [0023], [0043], [0045], [0076], [0088] | 4, 14 |
| A | claims, paragraphs [0023], [0043], [0045], [0076], [0088] | 3, 13 |
| X | 村山嘉延, 選択的単一胚移植(eSET)へ向けた体外受精卵の品質モニタリングシステムの開発, 科学研究費助成事業（科学研究費補助金）研究成果報告書 [オンライン], 31 July 2013, [retrieval date 09 December 2022], Internet: <URL: https://kaken.nii.ac.jp/ja/report/KAKENHI-PROJECT-20680026/20680026seika/> outline, section (4) | 1, 5, 6, 9, 11, 15, 16, 20 |
| Y | outline, section (4) | 4, 14 |
| A | outline, section (4), (MURAYAMA, Yoshinobu. Evaluation of the Embryo Quality for Elective Single Embryo Transfer. Final Research Report of Grants-in-Aid for Scientific Research (Scientific Research Grants) [online].) | 2, 3, 7, 8, 10, 12, 13, 17-19 |

☑ Further documents are listed in the continuation of Box C.    ☑ See patent family annex.

| | |
|---|---|
| *    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **09 December 2022** | **20 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/036466**

**C.    DOCUMENTS CONSIDERED TO BE RELEVANT**

| Category* | Citation of document, with indication, where appropriate, of the relevant passages | Relevant to claim No. |
|---|---|---|
| Y | TARGOSZ, Anna et al. Semantic segmentation of human oocyte images using deep neural networks. Biomedical Engineering Online. April 2021, vol. 20, no. 40, pp. 1-26<br>abstract | 4, 14 |
| A | abstract | 1-3, 5-13, 15-20 |
| Y | BARNETT-ITZHAKI, Zohar et al. Machine learning vs. classic statistics for the prediction of IVF outcomes, Journal of Assisted Reproduction and Genetics. 2020, vol. 37, pp. 2405-2412<br>abstract | 4, 14 |
| A | abstract | 1-3, 5-13, 15-20 |
| A | ITO, Hiroaki et al. Quantification of the Influence of Endotoxins on the Mechanics of Adult and Neonatal Red Blood Cells. J. Phys. Chem. B. 2015, vol. 119, pp. 7837-7845<br>abstract, section 2.4 | 1-20 |
| P, X | 山本暁久, 不妊治療をアシストする卵子の硬さ／柔らかさの定量評価技術, 新技術説明会, 株式会社TLO京都 京大技術移転部 [オンライン], 28 June 2022, [retrieval date 09 December 2022], Internet: <URL: https://shingi.jst.go.jp/pdf/2022/2022_kyoto-u_001.pdf> pp. 5-13, (YAMAMOTO, Akihisa), non-official translation (The hardness of eggs to assist sterility treatment/Softness quantitative evaluation technology of softness. New Technology Presentation Meetings! TLO-KYOTO CO., LTD. Department of Technology Transfer, Kyoto University [online].) | 1-20 |

Form PCT/ISA/210 (second sheet) (January 2015)

## INTERNATIONAL SEARCH REPORT
### Information on patent family members

International application No.

**PCT/JP2022/036466**

| Patent document cited in search report | Publication date (day/month/year) | Patent family member(s) | Publication date (day/month/year) |
|---|---|---|---|
| JP 2012-531584 A | 10 December 2012 | US 2012/0196316 A1 claims, paragraphs [0036], [0058], [0060], [0092], [0104] <br> WO 2010/151221 A1 <br> EP 2446251 A1 <br> CN 102460124 A | |

Form PCT/ISA/210 (patent family annex) (January 2015)

**REFERENCES CITED IN THE DESCRIPTION**

**Patent documents cited in the description**

- JP 2016054684 A

**Non-patent literature cited in the description**

- **LIVIA Z. YANEZ.** Human oocyte developmental potential is predicted by mechanical properties within hours after fertilization. *Nature Communications,* 2016, vol. 7 (1), 1-12
- *Force measurement in microenvironment using two-fingered micro hand (Arai Laboratory, Osaka University),* 22 January 2021, http://www.arail-ab.sys.es.osaka-u.ac.jp/index.php?option=com_content&view=article&id=75&Itemid=97&lang=ja>
- **MURAYAMA YOSHINOBU.** Elasticity measurement of zona pellucida using a micro tactile sensor to evaluate embryo quality. *Journal of Mammalian Ova Research,* 2008, vol. 25 (1), 8-16
- **ITO HIROAKI.** Quantification of the influence of endotoxins on the mechanics of adult and neonatal red blood cells. *The Journal of Physical Chemistry B,* 2015, vol. 119 (25), 7837-7845